Europäisches Patentamt

European Patent Office

Office européen des brevets

(11) Veröffentlichungsnummer: **0 369 292**
**A1**

(12) # EUROPÄISCHE PATENTANMELDUNG

(21) Anmeldenummer: **89120602.1**

(22) Anmeldetag: **07.11.89**

(51) Int. Cl.⁵: **C12Q 1/58**

(30) Priorität: **15.11.88 DE 8814264 U**

(43) Veröffentlichungstag der Anmeldung:
**23.05.90 Patentblatt 90/21**

(84) Benannte Vertragsstaaten:
**AT BE CH DE ES FR GB IT LI NL SE**

(71) Anmelder: **Röhm Pharma GmbH**
**Dr.-Otto-Röhm-Strasse 2-4**
**D-6108 Weiterstadt 1(DE)**

(72) Erfinder: **Rothgang, Gerhart**
**Im Röhrgewann 1**
**D-6109 Mühltal(DE)**
Erfinder: **Mann, Helmut-Josef**
**Im Häfer Feld 14**
**D-6086 Riedstadt(DE)**
Erfinder: **Klein, Cornelia J.**
**Tulpenweg 12**
**D-8755 Alzenau/Wasserlos(DE)**

(54) **Diagnostische Dosierungseinheit zur Ureasebestimmung.**

(57) Zur Ureasebestimmung dient eine wasserfreie Dosierungseinheit, vorzugsweise in Form eines festen Komprimats, die Harnstoff, eine Puffersubstanz für den Bereich pH 5,0 - 7,5 und einen pH-Indikator, der im pH-Bereich 5,5 - 8,5 einen Farbumschlag hat, in einer zur Ureasebestimmung geeigneten Menge enthält.

EP 0 369 292 A1

Die Erfindung betrifft ein diagnostisches Hilfsmittel in Form einer festen Dosierungseinheit zur Bestimmung von Urease. Die Zersetzung von Harnstoff zu Ammoniak und Kohlendioxyd durch das Enzym Urease wird analytisch sowohl zur Bestimmung von Harnstoff mittels Urease enthaltender Reagenzmischungen als auch zur Bestimmung von Urease mittels Harnstoff enthaltender Reagenzmischungen angewendet.

**Stand der Technik**

Die Bestimmung von Urease in Biopsaten der Magenschleimhaut hat Bedeutung bei der Diagnose von Magenerkrankungen, die durch Infektionen mit Campylobacter pyloridis verursacht sind. Die Anwesenheit dieses Bakteriums wird häufig diagnostiziert, indem man ein Biopsat der Magenschleimhaut in ein Harnstoff enthaltendes Kulturmedium einbringt und einen Farbindikator zum Nachweis von Ammoniak zusetzt. In diesem Falle stammt die Urease aus den im Kulturmedium gewachsenen Keimen. Ein für diese Bestimmung geeignetes Kulturmedium ist als Christensen-Harnstoffagar (vgl. W.B. Christensen, Journ. Bact.,52, S.461, 1946) bekannt.

Gemäß EP-A 204 438 wird eine wäßrige Reagenzmischung aus Harnstoff, einem Bactericid und einem Farbindikator zum Nachweis von Urease eingesetzt. Das Bactericid unterbindet jegliches Keimwachstum, so daß nur der Urease-Gehalt der untersuchten Probe festgestellt wird, jedoch nicht die erst im Testmedium durch eingebrachte ureasebildende Keime entwickelte Urease.

Die Handhabung der wäßrigen Reagenzmischung erweist sich jedoch in der Laborpraxis als verhältnismäßig aufwendig. Daher wurde in dem genannten Dokument vorgeschlagen, das wäßrige Medium durch gelbildende Zusätze, wie Agar, in einen halbfesten Zustand zu bringen. In dieser Form werden Dosierungseinheiten für Einzelbe-stimmungen in wasserdichte und verdunstungsge-schützte Packungen eingeschlossen. Wegen der hohen Anforderungen an die Verpackung können deren Kosten diejenigen für die Reagenzmischung selbst weit übertreffen.

Durch die Anwesenheit von Wasser wird allerdings der Harnstoff langsam zersetzt, wodurch Ammoniak freigesetzt und ein vorzeitiger Farbumschlag bewirkt wird. Um dies zu verhindern, enthalten die bekannten Präparate einen Puffer, welcher einen pH-Anstieg bei der Freisetzung von Ammoniak verhindert. Da der Urease-Nachweis aber gerade auf der Feststellung der Ammoniakfreisetzung beruht, muß die Menge des Puffers so bemessen werden, daß er zwar eine geringe Menge von hydrolytisch erzeugtem Ammoniak abfängt, aber bei einer größeren Menge Ammoniak, die beim Urease-Test gebildet wird, einen pH-Anstieg und einen Farbumschlag des Indikators zuläßt. Wird die Puffermenge niedrig bemessen, so ist die Pufferkapazität gering und die Lagerfähigkeit der Packung entsprechend begrenzt; nach Verbrauch des Puffers tritt Farbumschlag infolge der weitergehenden Harnstoffhydrolyse ein. Wird die Puffermenge höher bemessen, so fängt bei der Anwendung einer frisch bereiteten Testpackung der Puffer das durch Urease gebildete Ammoniak solange ab, bis er verbraucht ist. Erst danach kann ein Farbumschlag auftreten. Dadurch wird die Geschwindigkeit der Prüfung vermindert und vor allem ihre Empfindlichkeit deutlich verschlechtert.

**Aufgabe und Lösung**

Der Erfindung liegt die Aufgabe zugrunde, für die Urease-Bestimmung eine verbesserte Dosie-rungseinheit bereitzustellen, die für Einzelbestim-mungen leicht zu handhaben, einfach zu verpacken und unbegrenzt lagerfähig ist, eine hohe Empfindlichkeit hat und das Ergebnis schnell erkennen läßt.

Diese Ziele werden bei einer Dosierungseinheit zur Durchführung einer Ureasebestimmung mittels einer Reagenzmischung mit einem Gehalt an

    a) Harnstoff

    b) einer kleinen Menge einer Puffersubstanz für den Bereich pH 5,0 - 7,5,

    c) und einen pH-Indikator, der im pH-Bereich zwischen 5,5 und 8,5, jedoch oberhalb des Pufferbereichs einen Farbumschlag hat,

dadurch erreicht, daß die Dosierungseinheit frei von Wasser ist und wenigstens die Bestandteile a und b in einer zur Ureasebestimmung geeigneten Menge enthält. Die Dosismengen werden auf den Bedarf für eine Einzelbestimmung an einem Magenschleimhaut-Biopsat oder für eine definierte Mehrzahl solcher Bestimmungen bemessen.

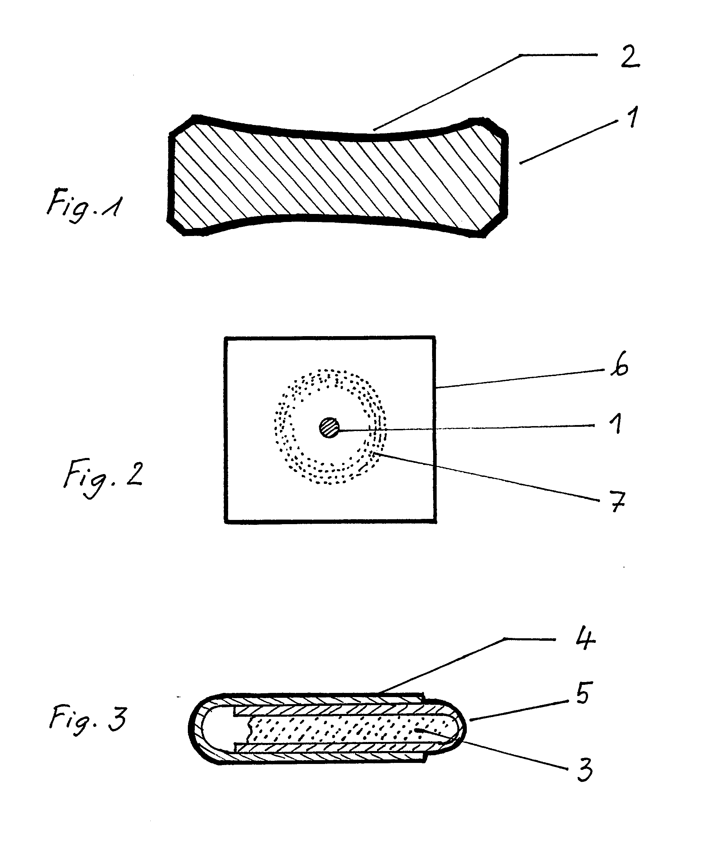

Zur besseren Erläuterung der Erfindung dienen die Figuren 1 und 2:

    **Figur 1** zeigt den Querschnitt durch eine erfindungsgemäße Dosierungseinheit in Form eines Komprimats im vergrößerten Maßstab.

    **Figur 2** stellt schematisch eine Prüfanordnung dar.

    **Figur 3** zeigt im vergrößerten Querschnitt eine mit der Reagenzmischung gefüllte Steckkapsel.

Wirkung und Vorteile der Erfindung

Die Dosierungseinheit ist dem Gebrauchszweck optimal angepaßt, da sie als abgeschlossene, feste Dosierungseinheit einfach zu handhaben und durch die Anwendung einfacher Herstellungsverfahren für pharmazeutische Dosierungseinheiten billig herstellbar ist. Vor allem ist durch die Abwesenheit von Wasser die Gefahr der Hydrolyse des Harnstoffs während der Lagerung ausgeschlossen. Ebenso ist eine Veränderung der Beschaffenheit durch Verdunsten von Wasser ausgeschlossen. Daraus ergibt sich eine nahezu unbegrenzte Lagerfähigkeit bei sachgerechten Lagerungsbedingungen. Durch einen verminderten Bedarf an Puffersubstanzen ist die Ureasebestimmung schneller, sicherer und genauer.

Die bevorzugte Ausführungsform der erfindungsgemäßen Dosierungseinheit ist die Form des Komprimats, das die trockenen Bestandteile der Reagenzmischung in verpreßter Form, in der Regel als Tablette, enthält. Sie können jedoch auch als Pulvermischung in geeignete Kapseln aus Hartgelatine oder einem anderen wasserlöslichen oder leicht zerstörbaren Werkstoff abgefüllt sein. Zur Verpackung der Dosierungseinheiten sind alle Verpackungsmittel, die üblicherweise für Tabletten, Kapseln und dergleichen verwendet werden, verwendbar, wie Fläschchen oder Röhrchen, Blisterpackungen oder folienversiegelte Prüfnäpfchen, in denen die Ureasebestimmung durchgeführt werden kann.

Zur Durchführung einer Ureasebestimmung mittels eines Komprimats wird das zu prüfende Biopsat auf das Komprimat aufgebracht und mit einem oder einigen Tropfen Wasser befeuchtet. Die in der Reagenzmischung enthaltene Puffersubstanz dient allein dazu, in dem wäßrigen Prüfmedium einen für die Reaktion günstigen pH-Wert von 5,0 bis 7,5, vorzugsweise 6,5 bis 7,0, einzustellen. Die dafür erforderliche Menge ist außerordentlich gering und beeinträchtigt weder die Schnelligkeit der Reaktion bis zum Eintritt des Farbumschlags noch die Empfindlichkeit.

Wird eine Dosierungseinheit verwendet, die die trockene Reagenzmischung in einer Kapsel enthält, so gibt man das Biopsat in eine kleine Menge Wasser und vermischt dieses mit dem Kapselinhalt. Bei wasserlöslichen Kapseln genügt es, die Kapsel in die wäßrige Prüflösung zu geben, wo sie sich auflöst und ihren Inhalt freigibt. Nicht oder schwer wasserlösliche Kapseln werden in der Prüflösung zerdrückt und dabei der Inhalt freigesetzt. Bei der Durchführung der Ureasebestimmung in einem Flüssigkeitsbehälter kann der pH-Indikator, falls er nicht in der Dosierungseinheit eingeschlossen war, als wäßrige Lösung zugetropft werden.

## Die Bestandteile der Reagenzmischung

Harnstoff wird vorzugsweise in einer Menge von 10 bis 320 mg pro Einzelbestimmung dosiert.

Als Puffersubstanzen eignen sich bekannte, in trockener Form lagerfähige Substanzen, deren Pufferbereich zwischen 5,0 und 7,5, vorzugsweise 6,5 bis 7,0, liegt. Geeignet sind z.B. Kaliumdihydrogenphosphat und Dinatriumhydrophosphat. Eine Menge von 0,01 bis 1 mg pro Dosierungseinheit ist in der Regel ausreichend. Mengen über 1 Gew.-% des Dosisgewichts sollten nicht verwendet werden, damit die Empfindlichkeit der Bestimmung nicht beeinträchtigt wird.

Der pH-Indikator soll einen deutlich wahrnehmbaren Farbumschlag oberhalb des Pufferbereichs, jedoch nicht oberhalb pH 9 zeigen. Geeignete Indikatoren sind nachfolgend aufgelistet, jeweils unter Angabe der Farben vor und nach dem Umschlag und der pH-Werte des Umschlagbereiches und - in Klammern - der Umschlaghalbwertstufe:

Bromkresolpurpur gelb/purpur 5,2/6,8 (6,12)
p-Nitrophenol farblos/gelb 5,0/7,0 (7,0)
Bromthymolblau gelb/blau 6,0/7,6 (7,07)
Phenolrot gelb/rot 6,8/8,4 (7,74)
Neutralrot 6,8/8,4
Chinolinblau 7,0/8,0
Kresolrot gelb/rot 7,2/8,8 (8,12)
m-Nitrophenol farblos/gelb (8,26)
m-Kresolpurpur gelb/purpur 7,4/9,0 (8,3)
Thymolblau gelb/blau 8,0/9,6 (8,89)

Mengen der Indikatoren von 0,001 bis 0,05 mg pro Dosierungseinheit sind in der Regel ausreichend. Bei der Herstellung von Komprimaten wird der Indikator in die Dosierungseinheit eingearbeitet, wenn der Farbumschlag auf der Oberfläche des Komprimats selbst beobachtet werden soll. Wird es dagegen bevorzugt, den Farbumschlag in einem getrennten Medium zu beobachten, z.B. in einer kleinen Wassermenge oder auf einem Filterpapier, so kann der Farbindikator auch darin enthalten sein.

Gemäß einer bevorzugten Ausführungsform enthält die Dosierungseinheit weiterhin ein keimtötendes Mittel, wie z.B. p-Hydroxybenzoesäureester. Es dient bei sterilen Reagenzmischungen der Unterdrückung des Keimwachstums nach der Inkubation und kann bei nicht sterilen Präparaten die Keimvermehrung während der Lagerung verhindern.

Gemäß einer weiteren bevorzugten Ausführungsform enthält die Reagenzmischung, wenn sie zu einem Komprimat verpreßt werden soll, einen inerten Extender. Er kann das 1- bis 50-fache Volumen der im Komprimat enthaltenen Bestandteile a bis c ausmachen und dient dazu, der für die Einzelbestimmung erforderlichen Menge der Bestandteile a, b und gegebenenfalls c eine leicht handhabbare Gestalt und Größe zu geben. Die genannten Bestandteile allein ergeben in der erforderlichen Menge eine sehr geringe Komprimatgröße, die sich weniger leicht handhaben läßt als ein Komprimat der angestrebten Menge von 20 bis 1000, vorzugsweise 50 bis 500 mg.

Der Extender ist als inert anzusehen, wenn er die Urease-Bestimmung nicht beeinträchtigt. Er darf vor allem den pH-Wert bei der Bestimmung nicht aus dem gewünschten Bereich bringen oder den pH-Anstieg während der Ureasebestimmung unterdrücken. Weiterhin darf er die Aktivität der Urease nicht herabsetzen oder selbst auf Harnstoff hydrolysierend wirken. Schließlich darf er keine Farbstoffe enthalten, die den Farbumschlag des Indikators nicht oder schwer erkennen lassen. Geeignet sind z.B. Natriumcarboxymethyl-Stärke, Cellulose und Celluloseabkömmlinge, wie Methylcellulose, Carboxymethylcellulose, Hydroxypropylcellulose, Hydroxypropylmethylcellulose, sowie anorganische unlösliche Substanzen, wie Calciumcarbonat oder kolloidales Siliciumdioxid.

Aufbau der Dosierungseinheit

Zur Herstellung von Komprimaten werden die Bestandteile der Reagenzmischung in der gebotenen Reinheit und mit geeigneter Teilchengröße gleichförmig vermischt und in an sich bekannter Weise zu Komprimaten 1 verpreßt. Ihre Form und Größe sollte für die Durchführung von Einzelbestimmungen von Biopsaten geeignet sein. Das Gewicht eines Komprimats liegt in der Regel unter 1 g, vorzugsweise unter 500 mg, beispielsweise zwischen 20 und 200 mg. Das bevorzugte Gewicht beträgt etwa 30 mg. Die Gewichtstoleranz ist hoch. Die Form sollte der Handhabung beim Aufbringen des Biopsats und einiger Wassertropfen entgegenkommen. Vorteilhaft sind flache runde Tabletten von 3 bis 25 mm Durchmesser, wobei die Oberflächen 2 leicht konkav gestaltet sein können.

Die Komprimate können für Schnelltests zum Screening von Biopsaten unsteril verpackt werden, werden jedoch zum Ausschluß von falsch positiven Ergebinssen vorzugsweise sterilisiert.

Die für eine Dosierungseinheit vorgesehene Menge der pulverförmigen Reagenzmischung 3 kann auch in eine Steckkapsel aus zwei Kapselhälften 4, 5 abgefüllt werden. Handelsübliche Steckkapseln aus Hartgelatine oder synthetischen wasserlöslichen Polymeren sind dafür geeignet. Da in diesem Fall die Mitverwendung von Extendern nicht sinnvoll ist, genügen sehr kleine Kapseln, beispielsweise von 5 bis 10 mm Größe. Es ist auch möglich, die Dosismengen zwischen zwei Metall- oder Kunststoffolien einzusiegeln oder einzuschweißen. Grundsätzlich ist jede Dosierungsform geeignet, aus der sich die trockene Reagenzmischung leicht in eine Prüflösung einbringen läßt.

Die Anwendung der Dosierungseinheit

Wenn alle Bestandteile a bis c der Reagenzmischung sowie gegebenenfalls ein Extender in einem Komprimat enthalten sind und das Biopsat auf dessen Oberfläche aufgebracht wird, tritt der Farbumschlag bei positivem Befund unmittelbar auf der Oberfläche des Komprimats in Erscheinung. Eine noch höhere Empfindlichkeit wird erreicht, wenn das Komprimat 1 während der Bestimmung auf einer trockenen, saugfähigen, dünnen Unterlage, z.B. einem Filterpapier 6, liegt, so daß das wäßrige Prüfmedium dort aufgesaugt wird und den mitgeführten pH-Indikator als farbigen Ring 7 um das Komprimat erkennen läßt. Bei dieser Art der Bestimmung braucht der pH-Indikator nicht im Komprimat selbst enthalten zu sein, sondern kann sich in der saugfähigen Unterlage befinden.

Die Urease-Bestimmung kann auch in einer etwas größeren Flüssigkeitsmenge vorgenommen werden, beispielsweise 0,5 bis 2 ml. In diesem Fall werden an die Gestalt der Dosierungseinheit keine weiteren Anforderungen gestellt, als daß sie leicht handhabbar ist und die enthaltene Reagenzmischung in der Prüfflüssigkeit schnell freigesetzt werden kann. Komprimate und gekapselte Dosierungseinheiten sind in gleicher Weise verwendbar. Der Farbumschlag bei Ureasegehalt ist dann in der Prüfflüssigkeit selbst erkennbar.

**Ansprüche**

1. Dosierungseinheit zur Durchführung einer Ureasebestimmung mittels einer Reagenzmischung mit einem Gehalt an
   a) Harnstoff
   b) einer Puffersubstanz für den Bereich pH 5,0 bis 7,5,
   c) und einem pH-Indikator, der im pH-Bereich zwischen 5,5 und 8,5, jedoch oberhalb des Pufferbereichs einen Farbumschlag hat,
dadurch gekennzeichnet
daß die Dosierungseinheit frei von Wasser ist und wenigstens die Bestandteile a und b in einer zur Ureasebestimmung geeigneten Menge enthält.

2. Dosierungseinheit nach Anspruch 1, dadurch gekennzeichnet, daß sie zusätzlich ein keimtötendes Mittel enthält.

3. Dosierungseinheit nach Anspruch 1 oder 2, dadurch gekennzeichnet, daß die Komponente c Bestandteil der Dosierungseinheit ist.

4. Dosierungseinheit nach einem oder mehreren der Ansprüche 1 bis 3, dadurch gekennzeichnet, daß sie die Form eines Komprimats hat.

5. Dosierungseinheit nach Anspruch 4, dadurch gekennzeichnet, daß sie einen inerten Extender enthält.

6. Dosierungseinheit nach einem oder mehreren der Ansprüche 1 bis 4, dadurch gekennzeichnet, daß sie in einem mit einem Verschluß versehenen Behälter dicht eingeschlossen ist.

7. Dosierungseinheit nach Anspruch 4, dadurch gekennzeichnet, daß das Komprimat ein Gewicht unter 1 g, vorzugsweise unter 500 mg hat.

Fig. 1

Fig. 2

Fig. 3

Europäisches
Patentamt

**EUROPÄISCHER RECHERCHENBERICHT**

Nummer der Anmeldung

EP 89 12 0602

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-4 101 382 (M.K. CHANG) <br> * Insgesamt * <br> --- | 1-3 | C 12 Q 1/58 |
| Y,D | EP-A-0 204 438 (B.J. MARSHALL) <br> * Seite 4, Zeile 4 - Seite 12, Zeile 3; Seite 14, Zeile 14, Seite 15, Zeile 5; Seite 16, Zeilen 15-24; Figuren 1-3 * <br> --- | 1-7 | |
| Y | US-A-3 527 674 (A. DEUTSCH) <br> * Spalte 2, Zeile 38 - Spalte 4, Zeile 38; Spalte 6, Zeilen 23-69; Spalte 8, Zeile 49 - Spalte 9, Zeile 70 * <br> ----- | 1-7 | |
| | | | **RECHERCHIERTE SACHGEBIETE (Int. Cl.5)** <br><br> C 12 Q |

Der vorliegende Recherchenbericht wurde für alle Patentansprüche erstellt

| Recherchenort | Abschlußdatum der Recherche | Prüfer |
|---|---|---|
| DEN HAAG | 05-02-1990 | HITCHEN C.E. |